# EUROPEAN PATENT SPECIFICATION

(11) **EP 1 601 784 B1**
(45) Date of publication and mention of the grant of the patent: **11.01.2012**
(21) Application number: 02806718.9
(22) Date of filing: 09.07.2002
(51) Int. Cl.: C12Q 1/68

(54) **MULTIPLE CONTROLS FOR MOLECULAR GENETIC ANALYSES**
MEHRFACHKONTROLLEN FÜR MOLEKULARE GENETISCHE ANALYSEN
TEMOINS MULTIPLES POUR ANALYSES GENETIQUES MOLECULAIRES

(30) Priority: 09.07.2001 US 303825 P
(43) Date of publication of application: 07.12.2005
(73) Proprietor: Children's Hospital Medical Center of Akron, Akron, OH 44308-1062 (US)
(72) Inventor: YAMIN, Moshe, Brighton, MA 02135 (US); WANG, Zhenywan, Sudbury, MA 01776 (US); MILUNSKY, Aubrey, Newton, MA 02459 (US); LEBO, Roger, V., Akron, OH 4304 (US)
(74) Representative: Patentanwälte Freischem
(86) International application number: PCT/US2002/021506
(87) International publication number: WO 2003/078570

(56) References cited:
- EP-A- 0 466 083
- EP-A2- 1 026 258
- WO-A-99/16904
- US-A- 5 834 181
- US-A- 5 834 181
- SHUBER A P ET AL: "EFFICIENT 12-MUTATION TESTING IN THE CFTR GENE: A GENERAL MODEL FOR COMPLEX MUTATION ANALYSIS" HUMAN MOLECULAR GENETICS, OXFORD UNIVERSITY PRESS, SURREY, GB, vol. 2, no. 2, 1 February 1993 (1993-02-01), pages 153-158, XP000335860 ISSN: 0964-6906
- SOCRANSKY S S ET AL: "CHECKERBOARD DNA-DNA HYBRIDIZATION" BIOTECHNIQUES, INFORMA LIFE SCIENCES PUBLISHING, WESTBOROUGH, MA, US, vol. 17, no. 4, 1 October 1994 (1994-10-01), pages 788-792, XP000474865 ISSN: 0736-6205
- BUYSE I M ET AL: "Diagnostic testing for Rett syndrome by DHPLC and direct sequencing analysis of the MECP2 gene: Identification of several novel mutations and polymorphisms" AMERICAN JOURNAL OF HUMAN GENETICS, AMERICAN SOCIETY OF HUMAN GENETICS, CHICAGO, IL, US, vol. 67, no. 6, December 2000 (2000-12), pages 1428-1436, XP002267501 ISSN: 0002-9297
- WILLIAMS L C ET AL: "Comparative semi-automated analysis of (CAG) repeats in the Huntington disease gene: use of internal standards" MOLECULAR AND CELLULAR PROBES, ACADEMIC PRESS, LONDON, GB, vol. 13, no. 4, August 1999 (1999-08), pages 283-289, XP004441557 ISSN: 0890-8508
- GILLES P N ET AL: "SINGLE NUCLEOTIDE POLYMORPHIC DISCRIMINATION BY AN ELECTRONIC DOT BLOT ASSAY ON SEMICONDUCTOR MICROCHIPS" NATURE BIOTECHNOLOGY, NATURE PUBLISHING GROUP, NEW YORK, NY, US, vol. 17, April 1999 (1999-04), pages 365-370, XP002928935 ISSN: 1087-0156
- SHUBER A P ET AL: "EFFICIENT 12-MUTATION TESTING IN THE CFTR GENE: A GENERAL MODEL FOR COMPLEX MUTATION ANALYSIS" HUMAN MOLECULAR GENETICS, OXFORD UNIVERSITY PRESS, SURREY, GB, vol. 2, no. 2, 1 February 1993 (1993-02-01), pages 153-158, XP000335860 ISSN: 0964-6906
- WILLIAMS L C ET AL: "Comparative semi-automated analysis of (CAG) repeats in the Huntington disease gene: use of internal standards" MOLECULAR AND CELLULAR PROBES, ACADEMIC PRESS, LONDON, GB, vol. 13, no. 4, August 1999 (1999-08), pages 283-289, XP004441557 ISSN: 0890-8508
- BUYSE INGE M ET AL: "Diagnostic testing for Rett syndrome by DHPLC and direct sequencing analysis of the MECP2 gene: Identification of several novel mutations and polymorphisms" AMERICAN JOURNAL OF HUMAN GENETICS, vol. 67, no. 6, December 2000 (2000-12), pages 1428-1436, XP002267501 ISSN: 0002-9297
- HO S N ET AL: "SITE-DIRECTED MUTAGENESIS BY OVERLAP EXTENSION USING THE POLYMERASE CHAIN REACTION" GENE, ELSEVIER BIOMEDICAL PRESS. AMSTERDAM, NL, vol. 77, no. 1, 1989, pages 51-59, XP000272761 ISSN: 0378-1119
- WANG ZHENYUAN ET AL: "Analysis by mass spectrometry of 100 cystic fibrosis gene mutations in 92 patients with congenital bilateral absence of the vas deferens" HUMAN REPRODUCTION (OXFORD), vol. 17, no. 8, August 2002 (2002-08), pages 2066-2072, XP002267502 ISSN: 0268-1161
- LEBO R V ET AL: "Rett syndrome from quintuple and triple deletions within the MECP2 deletion hotspot region.", CLINICAL GENETICS JUN 2001 LNKD- PUBMED:11453972, vol. 59, no. 6, June 2001 (2001-06), pages 406-417, ISSN: 0009-9163

## Description

### BACKGROUND OF THE INVENTION

Molecular genetic testing is becoming ever more important in prenatal diagnosis, maternal and newborn screening, screening for genetic disease in symptomatic and at-risk patients, identity and paternity testing, characterizing disease-causing organisms contracted from others or released by terrorists, characterizing recombinant genes in food, confirming the pedigrees of animals or plants, and identifying criminals. To conduct robust testing, reliable clinical laboratories are required by the College of American Pathologists to use normal and affected haplotype controls each time mutations or polymorphic sites are tested. A positive control is defined as extracted total DNA from an individual carrying an affected or polymorphic sequence. This DNA can be amplified and used directly as a test control or, in the case of mammals, cells can be transformed from individuals identified and sampled with the desired haplotypes so that an expanded supply of control DNA is available. However, a new test can be developed more rapidly than control nucleic acid samples from patients with abnormal allelic sequences can be obtained, as can be illustrated by the example of cystic fibrosis mutation testing in humans.

Cystic fibrosis gene testing began when mutations in the cystic fibrosis transmembrane receptor gene were reported to result in cystic fibrosis (Riordan et al., 1989; US Patent Nos. 5,776,677, 6,201,107, and 5,407,796). Over the next several years clinical cystic fibrosis testing for multiple mutations was offered by dozens of molecular genetics laboratories. These multiple PCR tests included amplifying fragments in which normal sequences could be distinguished from mutant sequences by differences in restriction enzyme sites, differences in the length of amplified products, or dot blot analysis with mutation-specific oligonucleotide probes. Controls were used that had been collected from patients with these individual mutations. Abnormal alleles from a single patient served as a control for each mutation specific test.

The need to acquire all required patient mutations sequences has become more immediate for cystic fibrosis testing even as other robust clinical tests using different methods have been under development. Currently, more than 1000 cystic fibrosis mutations and more than 50 polymorphic sites have been reported in the cystic fibrosis transmembrane receptor (cftr) gene. Twenty-five of these have been reported to occur at a frequency exceeding 0.1% (Grody et al., 2001). These 25 mutations have been designated to be included in testing panels that would meet the standard of care for screening pregnant Caucasian mothers (Grody et al., 2001). However, at this time, only 21 of these mutant alleles have been collected in cells that were transformed and are available through a central cell repository for purchase as positive controls. The remaining four mutant alleles are currently unavailable. Testing 25 mutations with only 21 mutant control sequences without simultaneously testing known mutant sequences from the remaining four sites decreases test reliability. A more reliable way of obtaining robust positive controls for all kinds of genetic tests is clearly desirable.

US-A-5 834 181 and Shuber, A. P., ET AL "EFFICIENT 12-MUTATION TESTING IN THE CFTR GENE: A GENERAL MODEL FOR COMPLEX MUTATION ANALYSIS" HUMAN MOLECULAR GENETICS, OXFORD UNIVERSITY PRESS, SURREY, GB, vol. 2, no. 2, 1 February 1993 (1993-02-01), pages 153-158, disclose methods for identifying genetic alterations in a target sequence present in a nucleic acid sample, comprising immobilizing samples on a support. The cited article by Shuber *et al*. discloses an efficient 12-mutation testing in the CFRT gene.

EP-A-1 026 258 discloses methods for generating polymorphic profiles for many polymorphic markers in many individuals in a population.

BUYSE, I. M., ET AL: "Diagnostic testing for Rett syndrome by DHPLC and direct sequencing analysis of the MECP2 gene: Identification of several novel mutations and polymorphisms" AMERICAN JOURNAL OF HUMAN GENETICS, AMERICAN SOCIETY OF HUMAN GENETICS, CHICAGO, IL, US, vol. 67, no. 6, December 2000 (2000-1 2), pages 1428-1436, describe methods for the identification of mutations and polymorphisms.

### SUMMARY OF THE INVENTION

The object of the present invention is solved by a molecular genetic testing method of incorporating three or more selected sequence changes from any genomic location into one or more selected target DNA sequences using template polynucleotides from one or many individuals, the method comprising the steps of:
synthesizing a positive test control by incorporating oligonucleotide primers comprising selected polynucleotide sequences into the target DNA sequence either by PCR amplification or site directed mutagenesis, and
assuring that all selected sequence changes, being mutations or polymorphisms, are present in the synthesized positive test control to minimize the number of control reactions or to simplify dispensing and selection of control aliquots in subsequent DNA assays.

The invention is directed to methods for genetic testing and to compositions comprising nucleic acid sequences for use as positive controls in any of the genetic testing methods of the invention. Compositions constructed according to the invention including multiple nucleic acid sequences are the optimal controls for simultaneously testing multiple variable nucleic acid sequences (i.e., mutations or polymorphisms) at one or more DNA or RNA sites in a test subject. Setting up a robust DNA test for multiple alleles and/or genomic sites requires the use of multiple controls that can be tested simultaneously to confirm that the assay conditions have been maintained in order to differentiate reliably among all tested alleles. With the use of compositions according to the invention, such controls can be synthesized and no longer need to be obtained from individuals with those sequences.

Oligonucleotide primers comprising selected polynucleotide sequences in the composition of the invention can be synthesized chemically and then extended by polymerase following hybridization to a longer complementary sequence within a cloned or extracted total genomic DNA sample. Extended primers are amplified by PCR or following cloning into selectable vectors that are introduced into a host such as bacteria or yeast for propagation. The amplified fragments are then purified, diluted to the correct concentration, and used as positive controls in a method according to the invention. Alternatively, the normal sequence to be changed to the mutated or polymorphic form can be cloned and then modified by site directed mutagenesis. Several single mutant or polymorphic sequences that together comprise a composition according to the invention, i.e. a panel of multiple control sequences, can be added individually to single site tests or mixed together or ligated together by further PCR or by cloning into vectors prior to use in individual or multiplex tests.

Thus, compositions of multiple controls can be used to minimize the number of control reactions in multiplex assays testing multiple sites or to simplify selection and dispensing of control aliquots in assays testing mutations or polymorphic sites individually. Controls according to the invention can be used when testing any genetically transmitted nucleic acid sequence. Tested nucleic acid can be derived from DNA and RNA viruses, nuclear DNA from single cellular and multicellular organisms, and inherited DNA in cellular organelles. Control compositions according to the invention can be used in tests such as determining the identity or classification of an individual derived from among any categorized organism; determining the genetic relationship of individuals to others within the same pedigree; screening populations for the prevalence of variants, mutations or other traits deemed worth testing; characterizing genetically transmitted diseases; and identifying and classifying strains of infectious diseases, e.g., contracted by accident or following intentional release. Controls according to the invention can also be used by organizations testing quality assurance and by companies maintaining quality control of manufactured genetic test kits.

The method according to the invention includes identifying one or more nucleic acid loci of the subject or subjects to be examined by the test to be conducted, identifying important multiple mutations or polymorphisms associated with the one or more selected loci, and planning and conducting the test, using as a positive control one or more nucleic acid fragments comprising three or more mutations or polymorphisms associated with the one or more selected loci. These control nucleic acid fragments can be used individually in separate, multiple tests or mixed together, or ligated together, for amplification by PCR or DNA cloning prior to simultaneous use in individual or multiplex tests. Positive controls synthesized according to the invention can be used when testing any genetically transmitted (heritable) nucleic acid sequence from any organism.

Kits are also provided to practice the present invention conveniently. These kits contain multiple allelic sequences as described herein in a single tube that can be aportioned and tested as controls within multiple single allelic tests or once as a single control for multiple allelic sites or alleles within a multiplex test. The kits contain, e.g., a vial with DNA sequences including two or more mutations or polymorphic controls that span the target locus or loci. The kits may also include a thermostable polymerase, other amplification reagents or restriction enzymes.

To aid in understanding the invention, several terms are defined below:
"PCR amplification reaction mixture" refers to an aqueous solution comprising the various reagents used to amplify a target nucleic acid. These include: enzymes, aqueous buffers, salts, target nucleic acid, and deoxyribonucleoside triphosphates. Depending upon the context, the mixture can be either a complete or incomplete amplification reaction mixture and the primers may be a single pair or nested primer pairs.
"PCR amplification reagents" refer to the various buffers, enzymes, primers, deoxyribonucleoside triphosphates (both conventional and unconventional), and primers used to perform the selected amplification procedure.
"Amplifying" or "Amplification," which typically refers to an "exponential" increase in target nucleic acid, is being used herein to describe both linear and exponential increases in the numbers of a select target sequence of nucleic acid.
"Bind(s) substantially" refers to complementary hybridization between an oligonucleotide and a target sequence and embraces minor mismatches that can be accommodated by reducing the stringency of the hybridization medium to achieve the desired priming for the PCR polymerases or detection of hybridization signal.
"Hybridizing" refers to the binding of two single stranded nucleic acids via complementary base pairing.
"Nucleic acid" refers to a deoxyribonucleotide or ribonucleotide polymer in either single- or double-stranded form, that unless otherwise limited also encompass known analogs of natural nucleotides that can function in a similar manner as naturally occurring nucleotides.
"Nucleotide polymerases" refers to enzymes able to catalyze the synthesis of DNA or RNA from a template strand using nucleoside triphosphate precursors. In the amplification reactions of this invention, the polymerases are template- dependent and typically add nucleotides to the 3'-end of the polymer being synthesized. It is most preferred that the DNA polymerase is thermostable for PCR amplification .

The term "oligonucleotide" refers to a molecule comprised of two or more deoxyribonucleotides or ribonucleotides, including primers, probes, nucleic acid fragments to be detected, and nucleic acid controls. The exact size of an oligonucleotide depends on many factors including its ultimate function or use. Oligonucleotides can be prepared by any suitable method including cloning and restriction enzyme digestion of appropriate sequences or direct chemical synthesis by a method such as the phosphotriester method of Narang et al., 1979, Meth. Enzymol. 68:90-99; the phosphodiester method of Brown et al., 1979, Meth. Enzymol. 68:109-151; the diethylphosphoramidite method of Beaucage et al., 1981, Tetrahedron Lett. 22:1859-1862; and the solid support method of U.S. Pat. No. 4,458,066.

The term "primer" refers to an oligonucleotide, whether natural or synthetic, capable of acting as a point of initiation of DNA synthesis under conditions in which synthesis of a primer extension product homologous to a nucleic acid strand is induced, i.e., in the presence of four different nucleoside triphosphates and an agent for polymerization (i.e. polymerase or reverse transcriptase) in an appropriate buffer and at a suitable temperature. A primer is preferably a single-stranded oligodeoxyribonucleotide. The appropriate length of a primer depends upon its intended use but typically ranges from 15 to 70 nucleotides. Shorter primer molecules generally require cooler temperatures to form sufficiently stable hybrid complexes with the template. A primer need not reflect the exact sequence of the template but must be sufficiently complementary to hybridize to a template.

The term "primer" may refer to more than one primer. For instance, if a region shows significant levels of polymorphism or mutation in a population, mixtures of primers can be prepared that will amplify alternate sequences. A primer can be labeled, if desired, by incorporating a label detectable by spectroscopic, photochemical, biochemical, immunochemical, or chemical means. Useful labels include p32, fluorescent dyes, electron-dense reagents, enzymes (as commonly used in an ELISA), biotin, or haptens and proteins for which secondary labeled antisera or monoclonal antibodies are available. A label can also be used to "capture" the primer, so as to facilitate the immobilization of either the primer or a primer extension product, such as amplified DNA on a solid support.

The term "reverse transcriptase" refers to an enzyme that catalyses the polymerization of deoxynucleoside triphosphates to form primer extension products that are complementary to a ribonucleic acid template. The enzyme initiates synthesis at the 3'-end of the primer and proceeds toward the 5'-end of the template until synthesis terminates. Examples of suitable polymerizing agents that convert the RNA target sequence into a complementary, DNA (cDNA) sequence are avian myeloblastosis virus reverse transcriptase and Thermus thermophilus DNA polymerase, a thermostable DNA polymerase with reverse transcriptase activity marketed by Perkin Elmer Cetus, Inc.

As used herein, the term "sample" refers to a collection of biological material from an organism containing nucleated cells. This biological material may be solid tissue as from a fresh or preserved organ or tissue sample or biopsy; blood or any blood constituents; bodily fluids such as amniotic fluid, peritoneal fluid, or interstitial fluid; cells from any time in gestation including an unfertilized ovum or fertilized embryo, preimplantation blastocysts, or any other sample with intact interphase nuclei or metaphase cells no matter what ploidy (how many chromosomes are present). The "sample" may contain compounds which are not naturally intermixed with the biological material such as preservatives anticoagulants, buffers, fixatives, nutrients, antibiotics, or the like.

The terms "allele-specific oligonucleotide" and "ASO" refers to oligonucleotides that have a sequence, called a "hybridizing region," exactly complementary to the sequence to be detected, typically sequences characteristic of a particular allele or variant, which under "sequence-specific, stringent hybridization conditions" will hybridize only to that exact complementary target sequence. Relaxing the stringency of the hybridizing conditions will allow sequence mismatches to be tolerated; the degree of mismatch tolerated can be controlled by suitable adjustment of the hybridization conditions. Depending on the sequences being analyzed, one or more allele-specific oligonucleotides may be employed. The terms "probe" and "ASO probe" are used interchangeably with ASO.

A "sequence specific to" a particular target nucleic acid sequence is a sequence unique to the isolate, that is, not shared by other previously characterized isolates. A probe containing a subsequence complementary to a sequence specific to a target nucleic acid sequence will typically not hybridize to the corresponding portion of the genome of other isolates under stringent conditions (e.g., washing the solid support in 2XSSC, 0.1% SDS at 70°C.).

"Subsequence" refers to a sequence of nucleic acids that comprise a part of a longer sequence of nucleic acids.

### BRIEF DESCRIPTION OF THE DRAWINGS

Other features and advantages of the invention will be apparent from the following description of the preferred embodiments thereof and from the claims, taken in conjunction with the accompanying drawings, in which:
Fig. 1A shows the preparation of PCR amplification constructs with two different immediately adjacent allelic sequences spanned by a single chemically synthesized polynucleotide that is used to prepare a longer genomic sequence for use as two positive control sequences in the method of the invention;
Fig. 1B shows PCR amplification to construct two or more site-specific allelic sequences within a PCR amplifiable sequence length of up to 20,000 basepairs for use as two positive control sequences in the method of the invention;
Fig. 1C shows splicing in any orientation of two PCR amplified fragments each with one or more allele-specific sites which may include restriction enzyme site(s) for use as two or more positive control sequences in the method of the invention;
Fig. 1D shows complementary ligation sequences such as but not limited to restriction enzyme site(s) added to the 5' prime terminus to facilitate subsequent unidirectional ligation for preparation of two or more positive control sequences according to the invention;
Fig. 1E shows the preparation of homozygous controls at two allelic sites for use as two positive controls in the method of the invention that provide sufficient flanking sequences in the 5' and 3' direction as required by the assay method and the length of the tested unknown genomic sequences;
Fig. 1F shows the preparation of heterozygous controls at two allelic sites for use as positive control sequences in the method of the invention;
Fig. 1G shows adding an additional homozygous mutation adjacent to two existing mutations synthesized as in Fig. 1E for use as three positive control sequences in the method of the invention;
Fig. 2 shows site directed mutagenesis with a single primer for preparation of one or more positive control sequences to be selected and used according to this invention;
Fig. 3 shows the preparation of four heterozygous positive control sequences according to this invention for use in cystic fibrosis testing ;
Fig. 4 shows mass spectrometry time of flight analysis of positive control sequences prepared as shown in Fig. 1F; and
Fig. 5 shows a cloning strategy for joining together 16 fragments that each contains one or more unique allelic sites for use as positive control sequences in the method of the invention.

### DETAILED DESCRIPTION OF THE INVENTION

Described herein are methods to optimally construct nucleic acid sequences for use as multiple positive controls in any desired genetic test format in which normal sequences are distinguished from mutant sequences and/or polymorphic sequences are distinguished from each other, for example, by determining differences in restriction enzyme sites, differences in the length of amplified products, or dot blot analysis with mutation-specific oligonucleotide probes. Other uses include as quality control standards in the manufacture of genetic tests, or as testing standards by regulatory agencies maintaining quality assurance. Compositions according to the invention, including multiple nucleic acid sequences constructed as described, are the optimal controls for simultaneously testing multiple variable nucleic acid sequences at one or more DNA and/or RNA sites. Setting up a robust DNA test for multiple alleles and/or genomic sites requires the use of multiple controls that are tested simultaneously to confirm that the assay conditions have been maintained so as to differentiate reliably among all tested alleles. With the use of compositions according to the invention, such controls can be synthesized and no longer need to be obtained individually.

Sequences according to the invention can be prepared chemically and/or by PCR amplification for use directly or after cloning and propagation. At the same time, some sequences can be PCR amplified and/or cloned directly from total genomic DNA obtained from an individual carrying the mutation or variant. Alternatively, the normal sequence to be changed can be cloned and then modified by site directed mutagenesis. Several single mutant or polymorphic sequences that together comprise a panel of multiple control sequences can be added individually to single site tests or mixed together or ligated together by further PCR or by cloning into vectors prior to use in individual or multiplex tests.

According to the method of this invention, in one embodiment any prepared DNA sequence with two or more abnormal or polymorphic sites associated with a desired genetic test can be used as a single control segment that is tested simultaneously with unknown samples. These can be represented by two or more adjacent sites within or between loci without regard to the actual location of these loci in the genome tested so long as the control sequence(s) spans the entire unknown sequences tested. For instance, two adjacent sites may be (1) within the same PCR primer site, (2) within any PCR amplifiable length, (3) within any cloned DNA sequence, (4) within any adjacent exons that were spliced and cloned from cDNA, (5) within the same exon, (6) in an exon and/or an adjacent intron, (7) in a gene promoter or enhancer region, (8) in one or more flanking gene regions, (9) at existing or new splice sites, (10) in any other two closely spaced adjacent sites, (11) in a selected cytogenetically or phenotypically important chromosome region(s), or (12) at sites comprising any combination of these categories. Sites may also be found on different chromosomes.

Adjacent regions of the same gene that are functionally significant to the DNA assay for the genetic test can be spliced together in any order to serve as multiple controls so long as the individual length of each spliced sequence is sufficient to serve as a robust control for the test applied. These cloned control segments that represent partial mutant gene sequences will have an analytical function that is derived from a previously characterized altered biological function, but the sequences in different fragments that are combined subsequently will be different spacially. Mutations may be found in any functional portion of a gene including exons, splice sites, and promoters as well as flanking gene regions. Variable di-, trior tetranucleotide repeat alleles may modify gene expression or function if the repeat number exceeds a disease-specific threshold or merely be used as very informative markers for identity or gene linkage studies. Frameshift mutations and destruction or insertion of a terminator codon also have a major impact on gene function.

In contrast, analytical function is defined by (1) the critical sequence length required for reliable testing, (2) a readily renewable source of a DNA sequence that can be prepared with high fidelity and validated before use that (3) can be used as a standard within and between laboratories to optimize test delivery. Other functionally useful analytical characteristics may include the most common mutations that represent the highest proportion of gene abnormalities in a patient population and the number and frequency of the remaining mutations or polymorphisms,

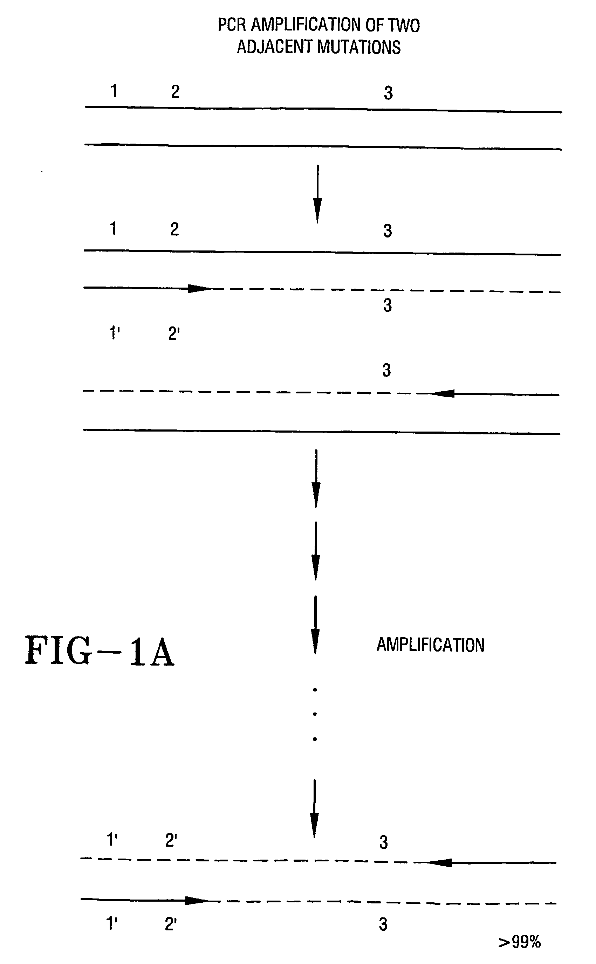

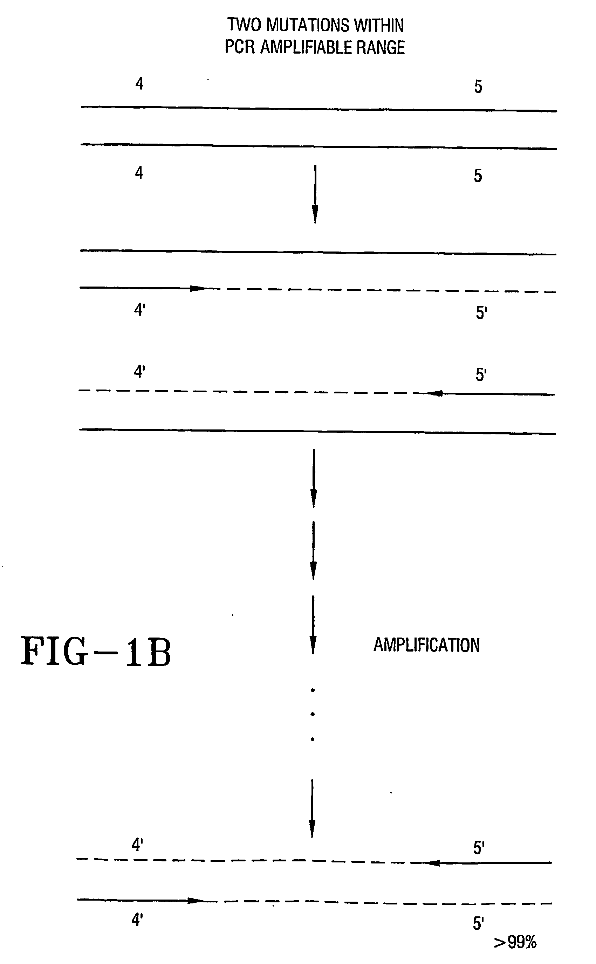

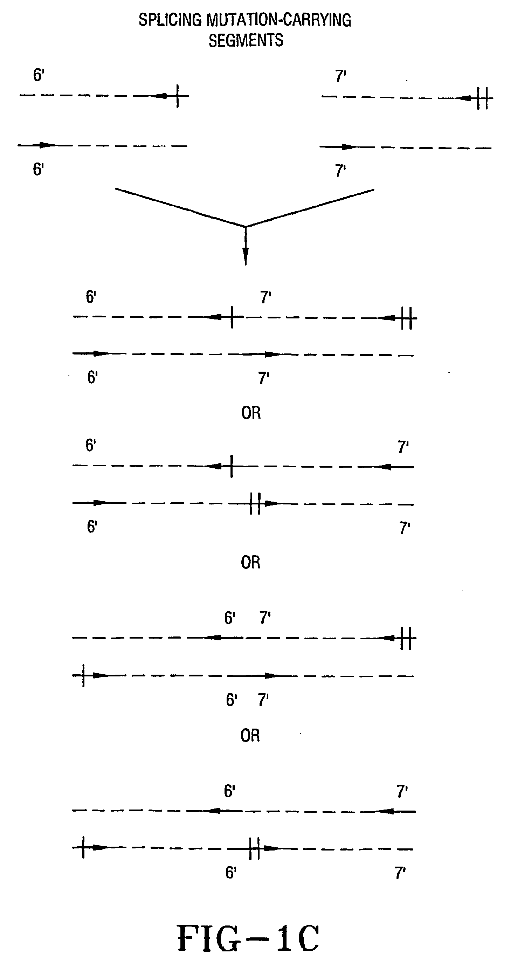

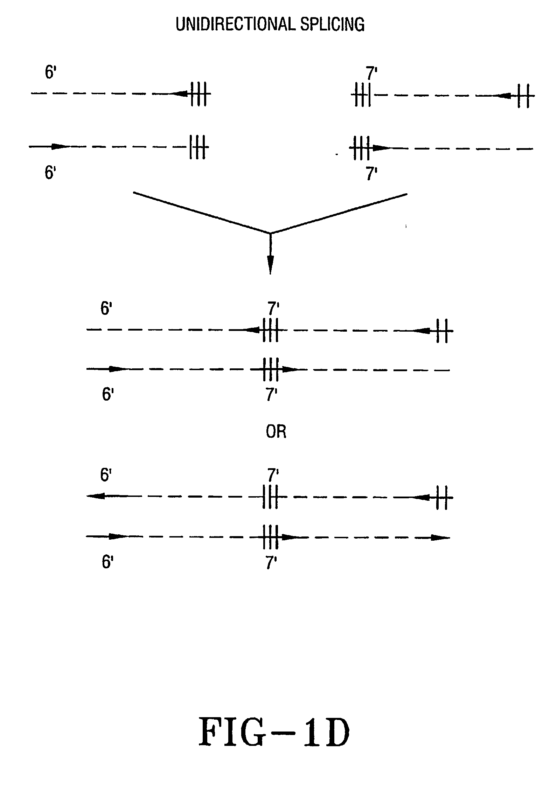

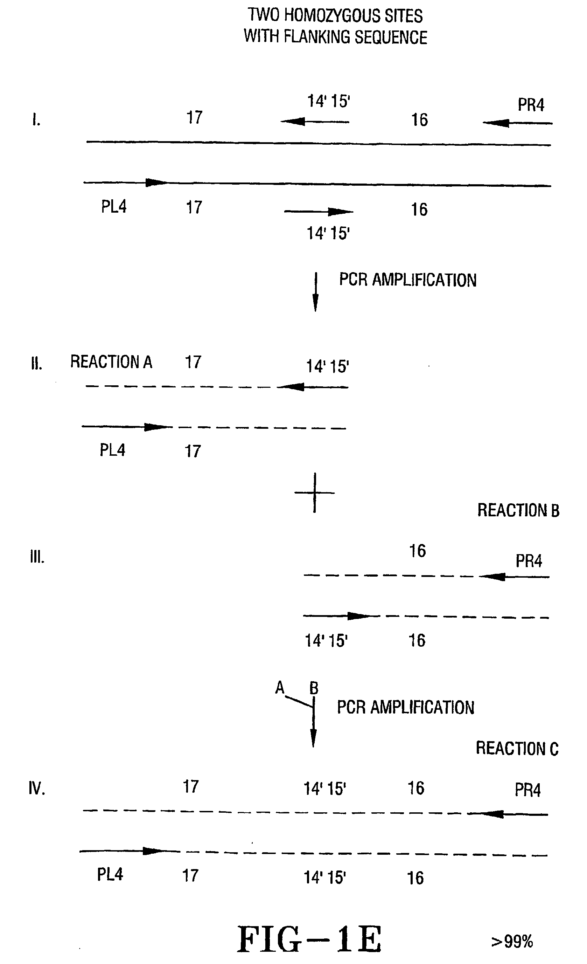

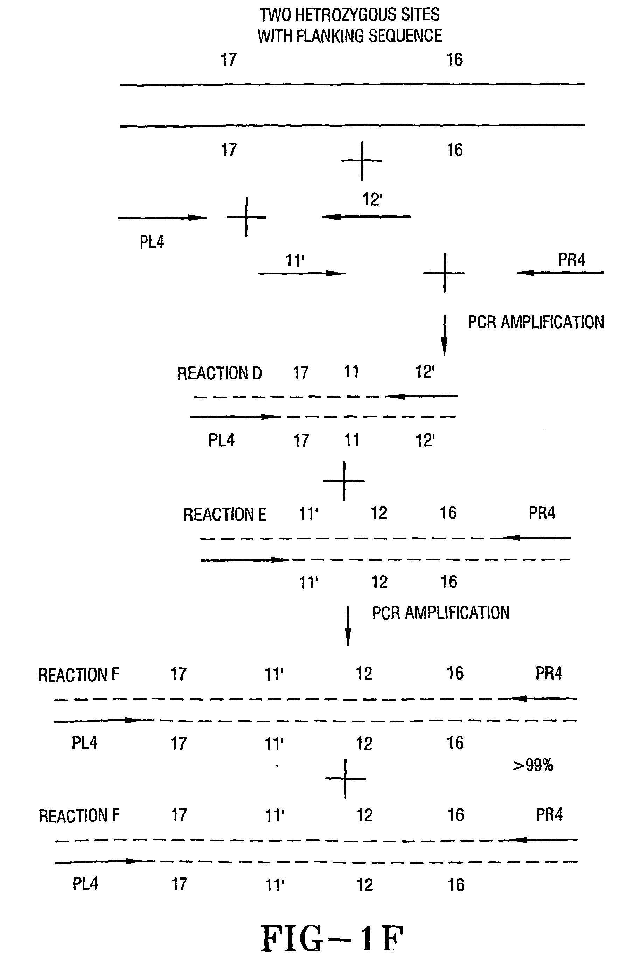

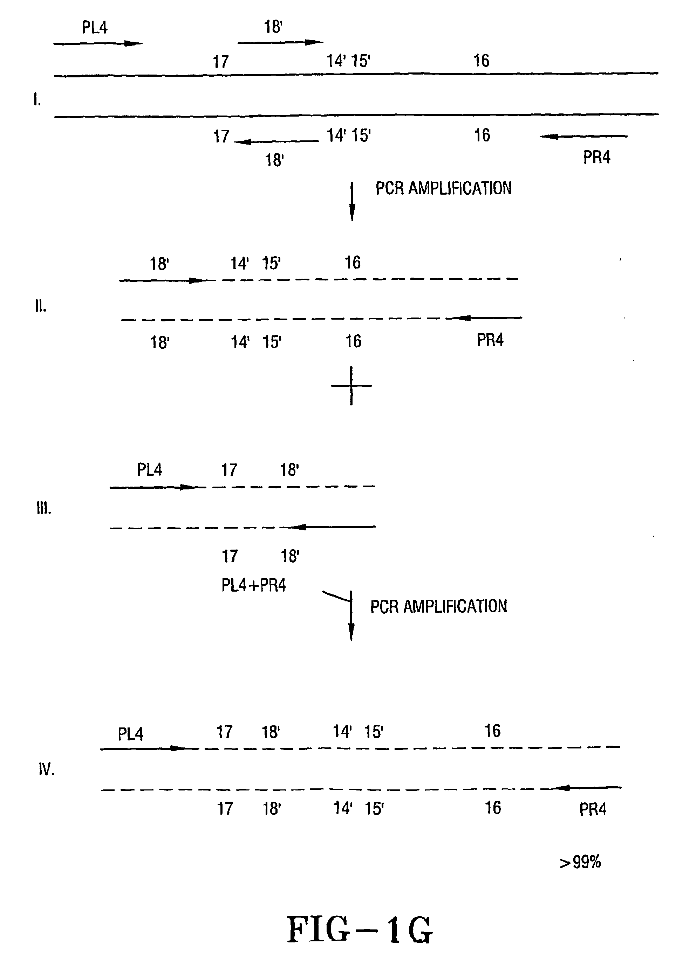

As will be described more fully in the Examples, Figs. 1A, 1B, and 1E demonstrate how to incorporate mutant sequences within a PCR amplifiable sequence length into single DNA fragments with or without normal flanking sequences by synthesizing site-specific PCR primers. Figs. 1C and 1D illustrate how individually synthesized primers can be spliced together. Fig. 1D further illustrates a ready means to direct splicing fragments in a preselected order and orientation. Fig. 1F illustrates how to synthesize heterozygous controls with both alleles in equal quantities within the same PCR amplification reaction. Fig. 1G teaches how to add one (18'; or more) control sequence adjacent to two existing control sequences (14' and 15'). The only limitation is that the additional modified DNA sequence (18') being introduced cannot be sufficiently close to another allelic sequence being tested (14' or 15') to interfere with the robust analysis of the preexisting control site.

Although some genes are very large, like the human dystrophin gene which spans >3,000,000 basepairs, only the length of functionally important gene modifications that can be measured readily by the test method are required to be spliced and placed together as a control. The limit of the lengths that can be spliced together and grown in large quantities in some living organism is defined by the capacity of the vector into which the fragments have been cloned.

Cloning strategies vary according to the vector's cloning site(s), the selectable gene markers at these cloning sites, and the capacity of the vector (Sambrook, Fritsch, & Maniatis, 1989). One efficient strategy for combining many different synthesized fragments would be to clone these into plasmids and, if need be, into phage or cosmids. The multiple cloning sites introduced into commercially available plasmid vectors like pUC19 have multiple restriction enzyme sites immediately adjacent to each other to simplify cloning strategy design.

These constructed vectors carrying multiple control sites would then be grown and isolated in an appropriate concentration to add to control tubes for PCR amplification adjacent to tubes with unknown total genomic DNA. The number of control DNA sequences per unit volume would be similar to total genomic target sequences in the unknown tubes.

Alternatively, a series of multiplexed PCR primers can be used to PCR amplify one, more, or all of the segments that are functionally significant to the test. It must be noted that continued PCR amplification of heterozygous stock PCR product might ultimately amplify one allele much more than the other even though the amplification efficiency differs by less than 3%. The method in Fig. 1F is designed to avoid this possibility and has been effective for all controls made to date. Nevertheless, this potential problem may be avoided further by making homozygous PCR products or cloning both fragments amplified at each site for continued propagation in bacteria.

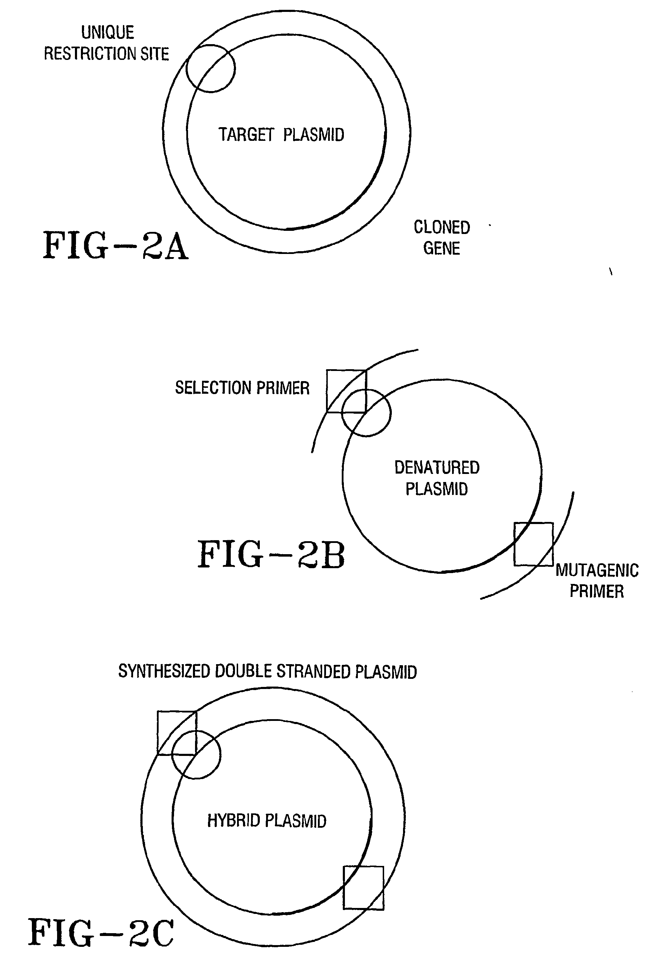

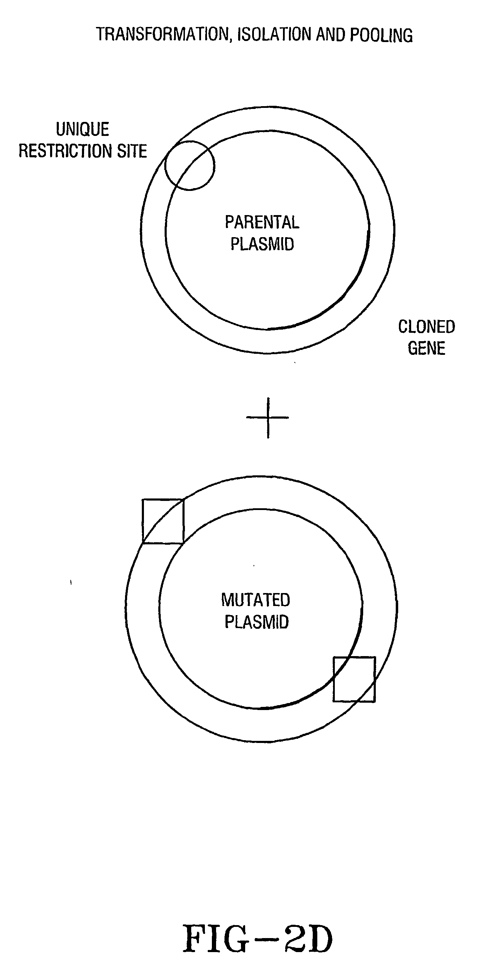

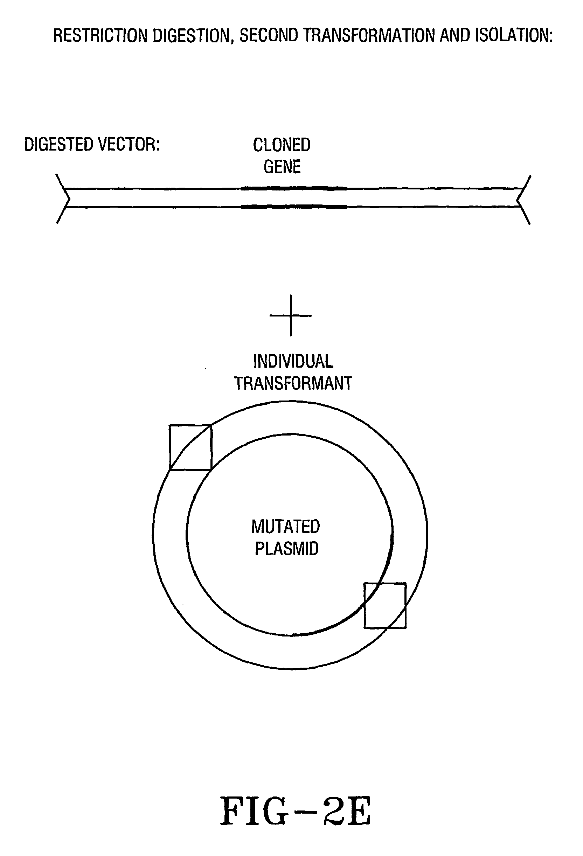

Yet another means to construct controls with multiple variant DNA sequences is to first clone or obtain a clone of the genomic region to be tested. Then, any mutation, polymorphism, or variant sequence desired can be introduced into the cloned segment by site directed mutagenesis, as shown in Fig. 2, or by the method described to introduce the 18' sequence in Fig. 1G. Using site directed mutagenesis, the mutagenic primer may have one or more mutant or polymorphic sites. One, two, or more mutagenic primers may be used simultaneously according to the method provided with the kit (Transformer Site-Directed Mutagenesis Kit, Clonetech Laboratories, Palo Alto, CA).

As more diseases need to be tested, more mutant sequences can be synthesized and added to the same DNA sample or to an additional tube with other multiple controls including tubes with very closely spaced DNA sequence changes that would interfere with each other's reliable analysis if both were tested together on the same DNA fragment. For instance, in addition to the cystic fibrosis gene, mutations can also be constructed for all designated mutations for other small and large genes that when mutated result in diseases like Rett Syndrome. Other tests include: (1) multiple genes that when mutated each result in the same genetic disease phenotype (like Charcot-Marie-Tooth Disease), (2) groups of genetic diseases commonly found in the same ethnic population (like Tay-Sachs disease, Gauchier disease, and Canavan disease in individuals of Ashkenazi Jewish descent and (3) the most common genetic diseases found in the general population (like Duchenne muscular dystrophy). Human screening alone encompasses testing women before and after conception as well as testing individuals during all stages of the life cycle from preimplantation embryos to geriatric patients. The same DNA construct carrying multiple abnormal and/or normal control sequences can be used to optimize sample testing and simplify assay design, set-up, and quality control for (1) multiple individual assays, (2) assays that test multiple locations simultaneously by unique length products measured with internal size controls as in gel electrophoresis or mass spectrometry, (3) multiplex assays that test several locations simultaneously as in testing multiple polymorphic loci with different colored products or on multiple microchip locations or (5) testing the absence, decreased, or increased quantity of a product.

Multiple DNA control segments that have been verified by sequencing can be used as a common standard by all testing laboratories to readily compare reliability within and between protocols and to optimize the interpretation of (1) mutation tests for disease genes like cystic fibrosis, (2) critical simple sequence repeat lengths like the fragments containing trinucleotide repeat numbers (i.e., 34 or 39 in Huntington disease), and (3) SNP (single nucleotide polymorphic) alleles also known as simple sequence repeat (SSR) alleles during identity testing, linkage analysis, or forensic testing. Constructing and using DNA fragments with multiple genetic mutations and polymorphisms as control sequences provides a ready means to determine whether all laboratories using the same protocols obtain the same results. For any one genetic disease, all mutations and polymorphic sites can be synthesized and tested. Alternatively, a subset of mutant, polymorphic and variant sites can be selected to identify a substantial proportion of mutations and/or the segregation thereof for any one genetic disease including the most common severe mutations, as has been recommended for cystic fibrosis. The mutations screened may be found in a group of diseases that are conveniently tested simultaneously because these genes are related technically, functionally, or are the most common in a selected target population. This embodiment is applicable to population screening as well as individual organism identification, classification, and infectious or genetic disease screening. Tested organisms include all single cellular and multicellular organisms at any stage of their life cycle.

Controls can be designed and synthesized to include all the optimal sequences required for mutation and/or polymorphic analyses at any locus including gene introns, exons, or flanking regions and polymorphic sites. Appropriate length artificially synthesized nucleic acid sequences from any organism can be designed to accommodate the specific testing format. Optimal controls can be synthesized with only abnormal sequences, i.e., homozygous controls, like those mutations found in both genes of individuals affected with autosomal recessive disease (Fig. 1G). Alternatively, controls can be synthesized with both a normal and an abnormal sequence (Fig. 1F), i.e., heterologous controls, like the mutations found in carriers of autosomal recessive disease and in different genes of individuals affected with an autosomal dominant disease.

Segments from more than one gene can be incorporated into the same control so that a single DNA segment can be used as the only control required in a multiple gene assay. These additional genes may be selected according to any useful criterion. Appropriate groups include genes in the same metabolic pathway that each may result in accumulation of a metabolite found in excess in the subject, or one of a family of genes that each contributes to a biologically active complex.

The methods described herein can be used for any DNA assay regardless of the organism from which the DNA is derived. Any organism that has ever been characterized and classified is included, from single to multicellular organisms, plants, and animals as well as their subcellular compartments with heritable DNA sequences, like mitochondria.

The following examples are presented to illustrate the advantages of the present invention and to assist one of ordinary skill in making and using the same. These examples are not intended in any way otherwise to limit the scope of the disclosure.

### EXAMPLE I

### PCR Amplification Constructs Immediately Adjacent Allelic Sequences

As shown in Fig. 1A, the sequences of two or more site-specific allelic nucleotide changes (1' and 2') can be synthesized directly into a single stranded DNA fragment that serves as an homologous PCR primer along with an homologous primer to the opposite strand spanning the downstream site. Even primers exceeding 60 basepairs long spanning more than one allelic site have served as unique PCR primers. The selected PCR primer pair can be used to amplify unique double stranded DNA products of a primer-defined length including normal and 1' and 2' nucleotide sequences using total DNA as an amplification template. Site 3 may represent one or more additional common allelic sequence(s) for which a different mutant allele is being prepared in a different segment. After multiple PCR amplification cycles, >99% of double stranded amplified PCR products of the correct length will include allelic sites 1', 2' and 3. (Any single site designation in this entire figure may also represent two or more variant locations .)

### EXAMPLE II

### PCR Amplification Constructs Two or More Site-Specific Alleles within a PCR Amplifiable Sequence Length

Referring to Fig. 1B, each of two individual primers of a complementary pair (4' and 5') can be synthesized to include one or more allelic sequences selected as a control. After multiple amplification cycles, >99% of the unique length double stranded fragments synthesized from this PCR primer pair will have both site-specific alleles. Any single primer may include two or more allelic nucleotide sequences within the single primer sequence. The minimum distance between two useful different allelic sequences in the same primer is determined by the DNA test method applied and the length and percentage of sequence homology in the DNA sequences that hybridize to the PCR product which together determine the melting temperature (Tm) required to denature hybridized double stranded DNA fragments. The specificity of hybridization and denaturation determines the reliability of the selected test method to distinguish between two different sequences in the subsequent assay.

### EXAMPLE III

### Splicing Together Two PCR Amplified Fragments Each with One or More Allele-specific Sites

Referring to Fig. 1C, fragments with two different allele-specific sites are ligated together to form a single unit that can serve as a control for both alleles. Nonspecific ligation like blunt ended ligation of two different fragments produces any one of four products. Any single site designation may represent two or more allele-specific sequences.

### EXAMPLE IV

### Complementary Ligation Sequences Added to the 5' Primer Terminus Facilitate Subsequent Unidirectional Ligation

As shown in Fig. 1D, primers that are constructed to have overlapping joining segments (illustrated with 3 vertical bars) in the first 6' amplification mix can prime another PCR amplified fragment with a complementary segment on one of the other primers used in the 7' amplification mix (Fig. 1D, top). Then the products can be digested with the same restriction enzyme selected so that digestion only occurs in the joining segments and digested fragments have single stranded complementary ends. Subsequently these complementary ends can be annealed and ligated unidirectionally to give the first product illustrated in Fig. 1D. Alternatively, given sufficient complementary sequence overlap at the 3' downstream location of the left fragment and the 5' upstream location of the right fragment, these two products (Fig. 1D, top) can be denatured, used to prime each other at location 7', and processed with polymerase to complete the synthesis of unique length double stranded longer products that have both the 6' and 7' allelic sequences ligated unidirectionally (Fig. 1D, bottom product). The number of different fragments that can be ligated to a unique location in any construct by this protocol is dependent upon the number of sequences that are required to assure unique non-homologous binding to one other fragment. This protocol provides sufficient capability to join any two fragments uniquely in any construct that can be envisioned. Site-specific joining sequences may be synthesized on the end of each primer so that multiple segments can be ligated and the final construct readily ligated into a vector for growth of commercial quantities.

### EXAMPLE V

### Homozygous Controls at Two Allelic Sites

Homozygous controls sufficiently close to the end of the required length of a PCR amplified product to be incorporated into an artificially synthesized PCR primer can be used to PCR amplify a homozygous control as in Fig. 1B. In contrast, when one or more homozygous control site(s) is required to be in the center of a longer length fragment, more than one approach can be used to synthesize this control sequence. (See, for example, Fig. 1E for the synthesis of a homozygous control and Fig. 1G for the synthesis of a heterozygous control). Fig. 1E shows that two complementary primers that include the variant sequence can be constructed that will subsequently bind to each other and serve as primers for PCR synthesis of one larger fragment with longer flanking sequences on both sides. The forward primer PL4 (Primer Left 4) along with the reverse primer with sites 14' and 15' amplifies the 5' double stranded DNA region in Reaction A. In a separate reaction tube the forward primer with sites 14' and 15' along with the reverse primer PR4 (Primer Right 4) amplify the 3' double stranded DNA region in reaction B. After these amplifications are complete, aliquots from reactions A and B are mixed, denatured, annealed, and the polymerase extends the products from reaction A and B that anneal at the 14' and 15' primer sites (Figure IE, IV). Now, additional primers PL4 and PR4 are added and PCR amplification continues, resulting in the synthesis of a product of which >99% is homozygous for sites 14', 15', 16 and 17.

### EXAMPLE VI

### Heterozygous Controls at Two Allelic Sites

As shown in Fig. 1F, primers PL4 and 12' together amplify a homozygous fragment with a variant sequence at site 12 and a common allelic sequence found in the total genomic DNA at site 11 (Fig. 1F, Reaction D). PCR primers 11' and PR4 amplify a homozygous fragment with a variant allelic sequence at primer site 11' and a common allelic sequence in the total genomic DNA being amplified at site 12 (Fig. 1F, Reaction E). These PCR reaction products can be synthesized and used independently as homozygous controls. These can also be mixed and PCR amplified as in Reaction F for use as full length heterozygous controls for sites 11' and 12' when sufficient flanking sequence is included in both products.

Products from Reactions 1 and 2 can also be ligated to each other as in Fig. 1C, grown in a vector or amplified again, and also used as heterozygous controls.

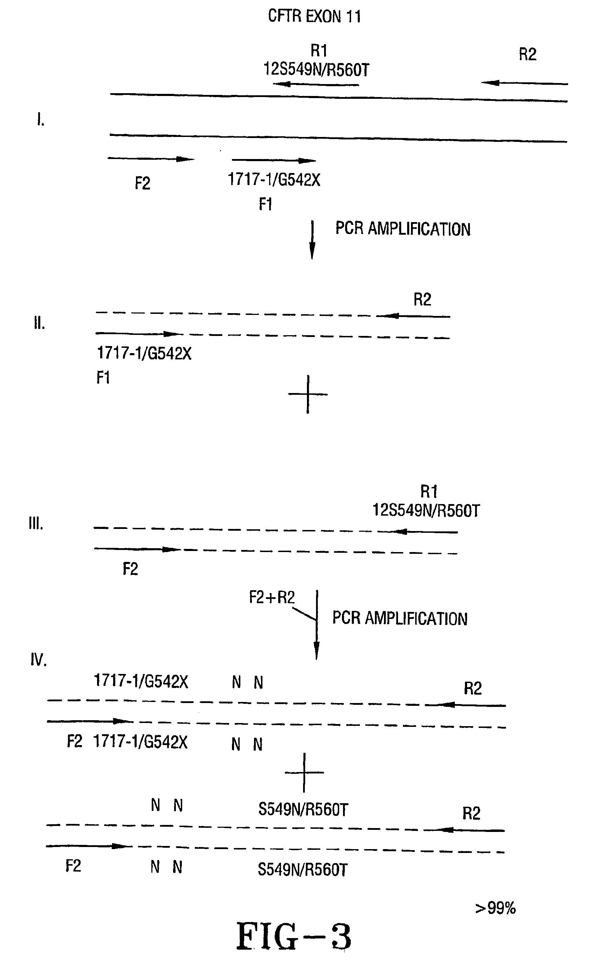

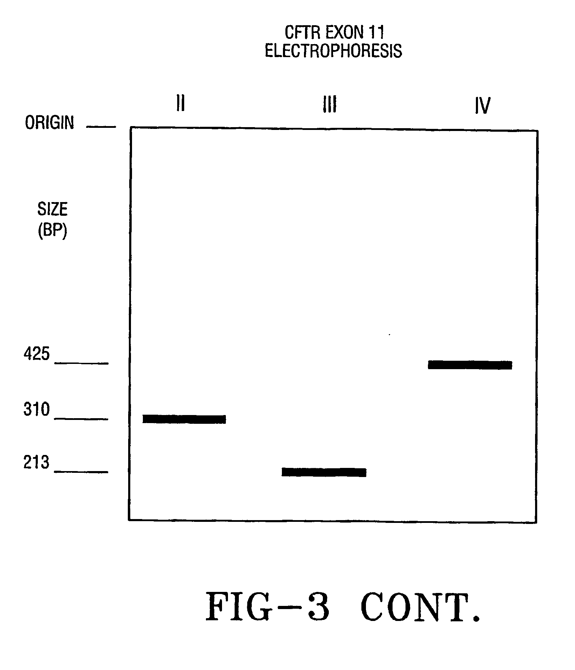

When control fragments are required that span the entire sequence from PL4 through PR4 that must include 11, 11', 12, and 12' sequences, these controls can be synthesized from Reactions 1 and 2 using additional PL4 and PR4 primers to synthesize a mixture of double stranded DNAs that are completely complementary in both strands and >99% of which span PL4 and PR4 (Fig. 1F, bottom). This product mixture is suitable for use as a heterozygous control for standard PCR assays with primers F1 and R2 followed by restriction enzyme analysis at both sites 11 and 12. This method was the first one enabled by PCR amplification and included the G542X, 1717-1G->A, S549N, and R560T loci These initial products were verified as heterozygous by both restriction enzyme analysis and by mass spectrometry (Fig. 3). (Note: Any single site designation may represent two or more locations of the same category. Any synthesized primer may have an extra segment on its 5' end for subsequent engineering.)

### EXAMPLE VII

### Adding Another Mutation adjacent to Two Existing Mutations

Referring to Fig. 1G, an existing DNA fragment with mutations at sites 14' and 15' is constructed as in Fig. 1E. Forward and reverse primers spanning the 18' mutation locus to be modified are selected, ordered and synthesized. PCR primers 18' and PR4 are used to amplify a homozygous fragment with a variant allelic sequence at primer site 14' and 15' and a common allelic sequence in the total genomic DNA being amplified at site 16 (Fig. 1G, Section II).

As shown in Fig. 1G, primers PL4 and 18' together amplify a homozygous fragment with a variant sequence at site 18 and a common allelic sequence found in the total genomic DNA at site 17 (Fig. 1G, Section III). Aliquots of both amplified fragments are added to another reaction tube with additional PL4 and PR4 primers, and PCR amplification continues until all the amplified fragments have mutations 18', 14' and 15' flanked by normal locations 17 and 16. The limit to this method is that a new mutation cannot interfere with the assay of an adjacent mutation. In that case, an additional fragment must be mutated for use either in the same or a separate control reaction mix. If the additional fragment is used in the same reaction mix, then that mix becomes heterozygous for the two mutations that were synthesized on separate fragments. Alternatively, the two PCR reaction products can be synthesized and used independently as homozygous controls. Any synthesized primer may have an extra segment on its 5' end for subsequent engineering, as in Fig. 1D.

### EXAMPLE VIII

### PCR Amplification Products of Heterozygous controls

This more complex example, illustrated in Fig. 1F, was enabled by constructing a mixture of two products carrying both the normal and mutant sequences at four CFTR gene sites: 1717-1,G->A; G542X; S549N; and R560T. PCR was used to amplify one 310 bp fragment with two mutations under the F2 primer site (1717-1 and G542X). In a separate reaction, a 213 bp fragment carrying two mutations in the R1 primer (S549N and R560T) were also amplified. These amplified fragments were confirmed to be the correct length as determined by agarose gel electrophoresis of the individual products. Then these two amplification products were mixed and further amplified with additional F1 and R1 primers to give a mixture of two new full length products each 425 bp long. Each product has two mutated sites and two normal sites. These products both exist in the reaction mix (IV) and co-migrate on agarose gel electrophoresis (Fig. 3, Lane IV).

Specifically, the first primer incorporates both the G542X and 1717-1 G->A mutation sequences: 11' (F1)'-AGA CAT CTC CAA GTT TGC AGA GAA AGA CAA TAT AGT TCT TTG AGA AAG. The second primer incorporates the S549N and R560T mutation sequences in the reverse complimentary strand direction: 12' (R1)-ACG TTG CTA AAG AAA TTC TTG CTC GTT GAC CTC CAT TCA GTG TGA. The 11'(F1) and 12'(R1) PCR primers can be used as a pair to amplify a single 98 bp sequence including all four mutations (Fig. 1B). After amplification of a homozygous 98 bp product, two additional primers (PL4(F2) and PR4(R2)) can be added to the synthesized fragment with total DNA to amplify a 425 bp fragment that spans the region carrying these four cystic fibrosis mutations: PL4(F2)-GAA GGA AGA TGT GCC TTT CAA and PR4(R2)-AGC AAA TGC TTG CTA GAC CA (Fig. 1E).

Alternatively, F1 and R2 can be used to amplify total DNA in one tube and F2 and R1 to amplify DNA in a second tube. This will amplify all synthesized products, half that carry the G542X and 1717-1G->A mutations and the other half that carry the S549N and R560T mutations (Fig. 1F, Fig. 3). Then, these sequences can be mixed together to amplify fragments with both normal and abnormal sequences at the same four loci in the same tube. If only the amplified fragments are used as primers to amplify full-length fragments from each other, then the resulting double stranded DNA fragments will be different from each other. These will serve as controls for ASOs and mass spectrometry. In order to use this as a control for restriction enzyme analysis, additional PCR amplification with PL4(F2) and PR4(R2) primers is required to produce completely complementary double stranded PCR products (Fig. 1F, bottom, Fig. 3-IV).

This PCR strategy was then used to synthesize 43 fragments carrying 93 mutations (Table 1), with 1 to 5 mutations included in the PCR amplified products at each of the 43 gene sites. Thus, no limitations have been encountered. At the same time, mutations in the same gene region disrupt the cftr gene function so that multiple mutations have been found in adjacent amino acid sites. Thus, among the 43 fragments synthesized, 4 fragments were synthesized with 5 mutation sites each. A fraction of these 43 PCR amplified fragments have been used as mass spectrometry controls on a routine basis.

### EXAMPLE IX

### Mass Spectrometry Time of Flight Analysis of Heterozygous Control

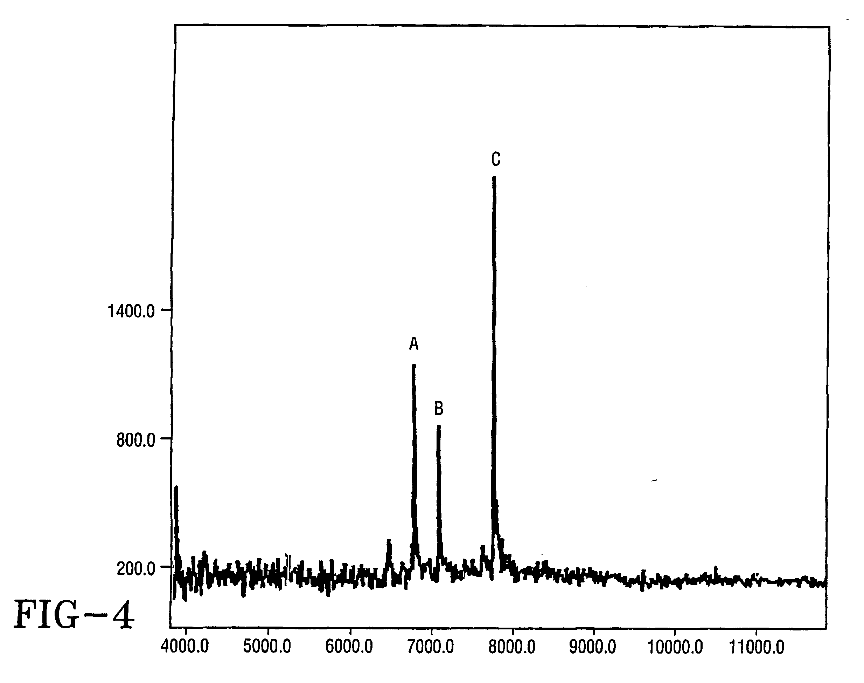

Analysis of product PL4-12' fragment (Example VI; Figure 3) used to amplify product 11'-PR4 (Figure 4) shows (A) the normal sequence and (B the synthesized mutant sequence at one selected control site along with (C) a longer normal sequence at an adjacent different mutation site in the DNA that was not modified in the PCR amplified product illustrated in Figre 3. All of the heterologous controls synthesized in Example VI have two peaks for each locus. Similar experiments were subsequently carried out for all mutations of Table 1, with similar results. This confirms that both normal and abnormal sequences are found at both locations.

**TABLE 1: Selected Examples of Synthesized CFTR Mutations.**

| | | | |
|---|---|---|---|
| 394delTT | 405+1G->A | G91R | 405+1G→A |
| 457TAT-G | I148T | Y122X | E92X |
| 574delA | 444delA | R117H | E21+1 |
| G178R | 711+5G→A | 711+1, G→T | 712-1,G→T |
| L206W, T→G | G330X | R374H | T338I |
| T360K | 1078delT | R334W | Q359K |
| T360K | R347P | I336K | R352Q |
| S354P | 1154insTC | 5T(delTT) | A455E |
| Q493X | DF508 | F520I | DF507 |
| G480L | V520F | 1609delCA | Q493R |
| 677delTA | C524X | R533X | P560K |
| G551D | R560T | 1717-1,G→A | G542X |
| Q552X | A559T | S549N | S549I |
| S549R | A561E | E585X | 1898+5,G→T |
| P574H | 1898+1,G→A, | 1812-1,G->A | Y563D |
| A49de194 | 2184delA | 2043delG | 2143delT |
| 2183AA->G | K710X | 2307 InsA2 | 184InsA |
| 2176InsC | 2789+5,G→A | Q890X | 5945L,C→T |
| 2869InsG | R1066C,C→T | W1089X,G→A | T1070Q |
| W1092X | R1158X | 3662delA | Q1238X |
| 3791delC | 3659delC | S1196X | R1162X |
| 3849+4,A->G | 3821delT | S1235R,T→G | 3849,G->A |
| D1270N,G→A | W1282X | 4005+1,G→A | W1282R |
| S1251N | | | |

| | | | |
|---|---|---|---|
| Note: All of these mutations were introduced so that both the normal and abnormal sequences were present in the amplified product. While this is the form of control that always tests any subsequent PCR to determine that both the abnormal and normal target sequences will amplify efficiently, only homozygous controls are required to meet all College of American Pathologists requirements. Therefore substituting only fragments with abnormal sequences would simplify the routine use of these multiple synthesized control DNA sequences. Whether homozygous, heterozygous or both types of controls are preferred depends upon the testing method. | | | |

### EXAMPLE X

### Cloning Strategy for 16 fragments

Cloning provides high fidelity biologically replicated sequence as a permanent renewable resource. Thus the controls can be relied upon when testing the reliability of the assay system. Aliquots of living organisms carrying the clone can be stored indefinitely to replace the growing stock in the event that a single basepair change occurs in a subsequent subculture. At the same time, the fidelity of this replication is confirmed by every assay for which the cloned sequences are used as a control.

Judicious primer sequence selection, enzyme digestion, annealing, cloning, selection, characterization, excision, and ligation to other cloned fragments provides a straightforward strategy to clone multiple control sequences into a single vector for subsequent propagation and use. To date more than 43 fragments with 1 to 5 mutations in each fragment have been synthesized. Given that the 92 mutations have been synthesized into 43 fragments that average 300 basepairs in length, a general strategy for cloning fragments with these characteristics follows.

Vectors with multiple cloning sites were developed in order to insert nearly any DNA fragment in the direction desired and to prevent ligation of the vector to itself to minimize background clones when searching for clones with inserted DNA. Directional cloning with different restriction enzyme sites on both sides of the cloning site is particularly useful to splice adjacent gene sequences together and to place sequences into protein expression vectors that need to express the sense strand. In contrast, the DNA fragments carrying the mutations merely require sufficiently long flanking sequence to be recognized by the assay. Unlike the actual gene sequence which must be intact to remain functional, the order and orientation of different synthesized fragments carrying mutations is unimportant so long as the sequences flanking each mutation are sufficiently long to be tested unambiguously by the assay employed. A restriction enzyme that cuts DNA at a recognizable 6 basepair restriction site will cut on the average of once every (4)⁶bp or 4096bp or 4.1kb. Very useful plasmid vectors on the order of 3 kb long are available with multiple cloning sites that are not found elsewhere in the vector's genome. Cloning sites typically have restriction fragment recognition sites of 6 basepairs or longer. Multiple cloning sites in selectable gene regions also typically have recognition sites of 6 bp or longer.

Small fragments are cloned first and then ligated to other fragments and cloned into another vector. As cloning of multiple fragments continues, the total fragment length becomes longer so that the vector category required to accommodate the insert may change. Plasmids can typically accommodate up to 10 kb of cloned DNA fragment, selected phage up to 25 kb of cloned insert, and cosmids up to 45 kb of inserted DNA.

The plasmid vector of choice will have a multiple cloning site and a readily selectable locus that when disrupted by a DNA insert can be distinguished from the phenotype when the vector is intact. One useful multiple cloning site and primer binding region in pUC18 and pUC19 has the same multiple cloning site in opposite directions in the center of the alpha-peptide of the beta-galactosidase gene. Disruption of this site produces colorless rather than blue colonies on medium containing ampicillin and X-gal. Therefor, recombinant clones can be distinguished readily from nonrecombinant clones.

A restriction enzyme site is then selected that is not found in the cloning vector or in the multiple cloning site. For instance, in the case of this illustration with pUC18 or pUC19, NotI (restriction site GCGGCCGC) is anticipated to cut once every 4^{8bp} = 65,536bp.

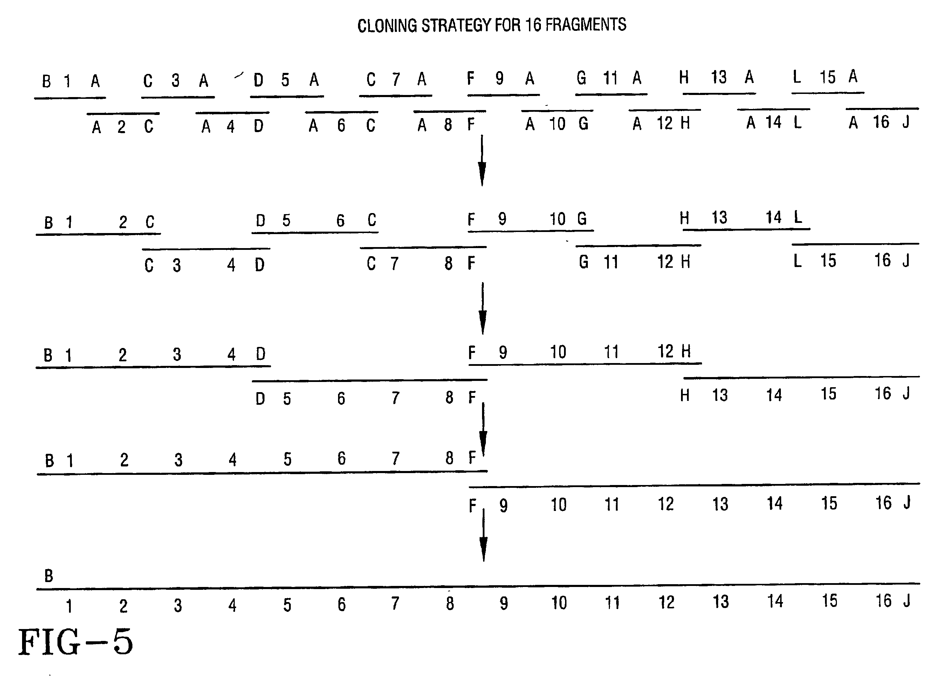

Then PCR primers are selected with restriction enzyme site sequences flanking the gene amplification sequences. For instance, if HindIII and SphI at locations 398 and 404 in the pUC18 vector are restriction sites b and c in Fig. 5, then the PCR primers are synthesized with the HindIII sequence flanking the forward PCR primer for fragment 1 and the NotI sequence flanking the reverse PCR primer for fragment 1. These primers will be used to PCR amplify fragment 1 from total genomic DNA. At the same time, the forward amplification primer for fragment 2 also has the NotI sequence at the 5' end and the fragment 2 reverse primer an SphI sequence at the 5' end. After PCR amplification of a unique gene target from total genomic DNA, the two fragments are then digested with NotI, HindIII, and SphI, and unincorporated primers, nucleotides, and restriction endonucleases are removed from both amplified fragments. After removal, the digested fragments 1 and 2 are ligated to each other at the NotI site and ligated into a pUC18 vector digested with HindIII and SphI. Recombinant clones are screened until colonies are found with fragments 1 and 2 ligated together. Then, one of these colonies can be grown up and the inserted fragment excised for subsequent cloning.

In an analogous fashion, fragments 3 and 4 are amplified from total genomic DNA with restriction site sequences SphI and NotI on fragment 3 and PstI and NotI on fragment 4 and cloned into another pUC18 vector at the SphI and PstI sites. This clone is then grown up, the insert excised and purified, and then fragment 1-2 is ligated at the SphI ("c") restriction enzyme site to fragment 3-4 (Fig. 5, line 2. After ligation to produce fragment 1-2-3-4, this fragment is subcloned into another pUC18 vector digested with HindIII and PstI (Fig. 5, line 3).

The same strategy is used to synthesize fragment 5-6-7-8 (Fig. 5, Lines 1,2,3). Then fragment 1-2-3-4 is ligated to 5-6-7-8 and subcloned into a HindIII(b) and XbaI(f) digested pUC18 vector. The same strategy is repeated until fragments 1-16 are subcloned into a single pUC18 vector at the HindIII and EcoRI sites. (As the PstI and SalI dual restriction enzyme digests work at very low efficiencies together, the SalI site will not be used.) Together these 16 fragments will yield a cloned insert about 4.8 kb long (300 bp X 16 fragments).

The 16 fragments that are cloned are selected so that none has an EcoRI or HindIII restriction enzyme sit. Then, this vector, with the 16 fragments, can be grown up and the 16 fragment insert excised intact for further cloning. Since the 43 fragments that average 300 basepairs are anticipated to span about 12,900 basepairs when placed end to end, this restriction site can be selected because it is not expected to occur within any of the 43 fragment sequences constructed. This enzyme selection would be designated "A" in Fig. 5. Then the 43 amplified heterozygous fragments with two fragments in each PCR amplified product would be organized into groups of up to 16 fragments with one or two PCR fragments for every amplified mutation locus. This would give six groups containing 86 PCR amplified fragments.

Additional control sequences can be added to the original plasmid by splicing in other prepared control sequences. Cloning multiple abnormal gene sequences into the same vector to be used as controls for multiple mutation tests merely requires that each abnormal sequence be present with sufficient flanking sequences to accommodate the requirements of the test design and test method employed including PCR amplification followed by restriction enzyme analysis, ASO, time-of-flight mass spectrometry, or direct or quantitative fluorescence analysis of unique fragments by multicolor fluorescence analysis.

Even though the order of the cloned gene fragments is generally unimportant for synthesized DNA sequence controls, multiple fragments can be cloned and ordered as desired within single vectors with multiple cloning sites for ease in construction. For instance, pUC18 and pUC19 plasmids have a multiple cloning site with multiple restriction enzyme sites listed in order: (b)HindIII, (c)SphI, Sse8387, (d)PstI, HincII, (e)XbaI, (f)BamHI, (g) SmaI, (h)KpnI, (i)SstI, and (j) EcoRI. If the unidirectional cloning site designated by vertical bars in Fig. 1D is recognized by a restriction enzyme with an 8 basepair specificity like NotI, which cuts GC/GGCCGC sequences (Enzyme "a" in Fig. 5), then fragment 1 can be spliced with fragment 2, segment 3 with fragment 4, segment 5 with segment 6, etc. to convert the 16 different fragments (Fig. 5, Line 1) into 8 spliced fragments in 8 different reaction tubes (Fig. 5, Line 2). These 8 fragments can each be subcloned into pUC18 at the multiple cloning site digested with two enzymes along with the fragment spliced at restriction site "a." Thus fragment 1,2 is cloned into the b-c(HindIII-SphI) cloning site and fragment 3-4 is cloned into the c-d(SphI-PstI) cloning site. These are grown in sufficient quantity to confirm the cloned fragments are the correct sequences in the predetermined order in the multiple cloning site. Then, these cloned segments are excised from the vector by digestion with the two enzymes sites used to clone the fragment into the vector, the insert from the vector is purified, and fragment 1-2 is ligated to fragment 3-4 at the c(SphI) restriction site (Fig. 5, Line 3). At the same time fragments 5-6-7-8, 9-10-11-12, and 13-14-15-16 are also ligated by an analogous strategy. Then, pUC18 is digested with enzymes b(HindIII) and d(PstI), purified, and ligated to fragment 1-2-3-4 at these two sites. This strategy produces a pUC18 vector that has a cloned 1-2-3-4 fragment. Again, the remaining fragments 5-6-7-8, 9-10-11-12, and 13-14-15-16 are ligated to a pUC18 vector digested with the two appropriate enzymes. These ligated fragments are cloned into bacteria and grown up as before to produce sufficient quantities of vector plus insert for the subsequent cloning step. Cloned fragment 1-2-3-4 will be excised from its vector using restriction enzymes b and d, separated from the vector, and ligated to excised fragment 5-6-7-8 from another clone (Fig. 5, lines 3-4). By repeating this strategy as drawn, 16 fragments can be ligated together into a single fragment flanked by b(HindIII)-j(EcoRI) restriction enzyme sites that can be used to cut this fragment only at the ends and the pUC18 vector at the multiple cloning site only and clone the two together. The only prior planning required is to determine that any fragment for which a restriction enzyme is subsequently used to splice that fragment from the vector does not contain that restriction site. For instance, synthesized fragment 1 cannot have b, c, d, f, or j restriction sites within its sequence. Otherwise, subsequent digestion with any of these restriction enzymes used to construct the long contig would also cut the fragment into smaller units. At the same time, fragment 12 should not be cut by restriction enzymes h, g, f, and j anywhere but at the end sites digested for subsequent cloning. This requirement is not anticipated to interfere with cloning because all of the restriction enzyme sites in the multiple cloning site recognize 6 or 8 basepairs. A restriction enzyme that recognizes a 6 basepair sequence is anticipated to cut on the average every (4)⁶ or 1024 basepairs. The average cloned fragment with cystic fibrosis mutations is 300 basepairs. Therefore an enzyme that recognizes a 6 basepair site is anticipated to cut only once in about 1 of 13 synthesized fragments. For a fragment that is cut by restriction enzyme b or c, another restriction enzyme cascade like f, g, h, and j are anticipated to be appropriate substitutes for cloning into a final contig of fragments 1-16. To date, 43 fragments have been synthesized with cystic fibrosis mutations. These are anticipated to be cloned readily into three different plasmid vectors. The order in which the restriction sites are used can be modified to accommodate all fragment sequences. Furthermore, other vectors like pBR322 can be substituted for pUC18 as required because this vector also has other cloning sites including 7 cloning sites found within the tetracycline resistance gene and several other sites flanking this gene. Upon growing these plasmid vectors with inserts to sufficient quantity for routine analysis as controls, these could be digested with enzyme selected because it does not cut within the 16 cloned fragment sequences. This would provide linearized cloned fragments with multiple cloned fragments ready for analysis.

The strategy used to ligate and clone 16 fragments would give a total mutation-carrying fragment of about 4.8 kb if each fragment were approximately 300 bp. In order to splice together 2 or more of these fragments, different restriction sites can be used within pUC18 or in other plasmid vectors like pBR322 that hold up to 10 kb readily. Two different synthesized fragments with 16 contiguous control sequences could be excised at two different enzyme sites like HindIII and EcoRI, spliced together at the HindIII site, and cloned into another plasmid vector at the EcoRI site because these vectors are reported to hold up to 10 kb readily. This description should not be construed to exclude using other vectors with cloning sites that modify selectable gene markers that can be used just as effectively as pUC18 or pBR322. Furthermore, a multiple cloning site with rare restriction enzyme cutting sites may be synthesized and used with much less regard for the sequences in each synthesized control sequence.

The next step requires ligating two fragments and cloning these into a plasmid, or phage. Then two or three different phage inserts can be introduced into cosmids. Judicious selection of restriction enzyme cloning sites can accomplish this goal. For instance, plasmid vectors can be induced to accommodate up to 25 kb but at a much lower efficiency like the 25 kb cloned McArdle gene sequence used for gene mapping (Lebo et al., 1984). Phage vectors which hold up to 22 kb, and cosmid vectors hold about 40 kb. The protocols for growing each of these vectors differs (Maniatis, 1982; Sambrook et al., 1989). Each has been well characterized and has been used readily for cloning with high efficiencies, even starting with less than 5 micrograms DNA extracted from flow sorted human chromosomes (Lebo et al., 1986). Then, large quantities of the cloned insert can be grown, either as suspension bacterial cultures (plasmids and cosmids) or plaques (phage) for preparation of large quantities of insert for use either as controls or for ligating to other cloned control fragments into a vector that accommodates even larger inserts like BACs or YACS.

### USE

Obtaining these allelic sequences in total DNAs from individuals in the population is seldom possible without prior extensive testing of many individuals. In the end, screening may still fail to identify all of the different control alleles. Nevertheless, agencies regulating molecular genetic laboratories have required that controls be included within each genetic test. In contrast to prior art control protocols, for which each cell line must be grown and the DNA extracted prior to use in multiple assays in order to test all available controls, compositions of positive control sequences according to the invention are easy to prepare and afford multiple advantages. For example, cloning and propagating all DNA control sequences synthesized removes the concern for adding PCR amplified products at the beginning of a PCR assay where the smallest aerosol droplet that finds its way into an assay tube with an sample of unknown genotype will change the test result. Additionally, the overall experimental design of a genetic test is simplified substantially by using controls with multiple allelic sequences according to this invention because multiple controls either minimize the number of control assays required in multiplex tests or simplify control sample addition to multiple tubes.

PCR is an extremely sensitive and rapid means to analyze DNA and is used whenever possible to enrich the sequence to be analyzed about 1,000,000-fold in standard analyses and about 1,000,000,000-fold in single cell analyses so the target sequences can be assayed more readily. Thus, PCR is used for about 80% of the molecular genetic analyses in diagnostic laboratories and is applied routinely in forensic and identity testing. Since many tests are based upon PCR amplification, total genomic DNA controls have been preferred or required by regulatory agencies in the past over PCR amplified controls because PCR amplified controls have so many amplified copies that a single droplet of aerosol that travels from the PCR amplified DNA into another tube with an untested sample can change the result of the sample test. However, the Molecular Genetics Committee of the College of American Pathology has agreed unanimously to allow the use of artificially synthesized controls prepared according the method of the invention. Any mutant or polymorphic allelic sequence that is required as a control to conduct a robust DNA analysis can be synthesized and subsequently cloned whenever desired according to the methods of the invention. Thus, synthesized controls can be used to test any genetically transmitted nucleic acid sequence, e.g., from the nucleus or from an organelle, from any organism.

Following the methods taught herein, PCR can be used to synthesize many copies of any desired abnormal sequence from well selected short primers that are synthesized artificially and used to PCR amplify short abnormal sequences into longer normal gene sequences found in total genomic DNA in many individuals of the species being tested. These PCR amplified products can be added later in any assay as a control. At the same time, these products can be cloned together into a single DNA fragment with multiple segments that can be propagated biologically in a single cell organism like bacteria or yeast, isolated, and used at the same target DNA sequence number per unit volume. Whenever desired, total genomic DNA from an unrelated species can be added to the control DNAs so that the total number of nucleotides per unit volume is identical to the number of nucleotides in the uncharacterized genomic DNA samples being tested. With one or both of these modifications, the multiple control sample can be added at the beginning of any assay, including a PCR amplification assay, at the same time as a total genomic DNA sample with an unknown genotype is being tested.

Additionally, the overall experimental design of a genetic test is simplified substantially by using controls with multiple allelic sequences according to this invention because multiple controls either minimize the number of control assays required in multiplex tests or simplify control sample addition to multiple tubes. For example, some currently available formats for the 25 mutation cystic fibrosis test analyze all 25 locations in genomic DNA simultaneously (Roche). A control that has all 25 mutation sites in the same DNA fragment constructed according to this invention can be used once in each multiplex assay to determine simultaneously whether the assay is robust at all 25 sites, saving substantial cost and analysis time. In other formats, the assay requires individual tests for each mutant or polymorphic locus. A control according to the invention as above can still be used, i.e., the same control can be added with each site tested so that assay design and analysis is simplified.

While the present invention has been described in conjunction with a preferred embodiment, one of ordinary skill, after reading the foregoing specification, will be able to effect various changes, substitutions of equivalents, and other alterations to the compositions and methods set forth herein. It is therefore intended that the protection granted by Letters Patent hereon be limited only by the definitions contained in the appended claims and equivalents thereof.

### REFERENCES:

Methods of detecting cystic fibrosis gene by nucleic acid hybridization. US5776677.
Cutting et al., The Johns Hopkins University Cystic Fibrosis Mutation Cluster. US5407796.
Grody WW, Cutting GR, Klinger KW, Richards CS, Watson MS, Desnick RJ. Laboratory standards and guidelines for population based cystic fibrosis carrier screening. Genetics in Medicine 3(2):149-154, 2001.
Gusella JF, MacDonald ME. Trinucleotide instability: a repeating theme in human inherited disorders. Annual Review Medicine 47:201-209, 1996.
Hummerich H, Baxendale S., Mott R., Ikirby SF, MacDonald ME, Gsella J, Lehrach H, Bates GP. Distribution of trinucleotide repeat sequences across a 2Mbop region containing the Huntinton's disease gene. Hum Mol Genet 3(1):73-78, 1994.
Lebo RV, Ikuta, T, Milunsky JM, Milunsky A. Rett Syndrome from quintuple and triple deletions within the MECP2 deletion hotspot region. Clinical Genetics, 59:406-417, 2001.
Lebo R, Maher T, Farrer L, Yosunkaya Fenerci E, Milunsky J. Highly polymorphic short tandem repeat analyses clarify complex molecular test results. Diagnostic Molecular Pathology, 10(3):179-189, 2001.
Lebo RV, Gorin F, Fletterick RJ, Kao F-T, Cheung MC, Bruce BD, Kan YW: High-resolution chromosome sorting and DNA spot-blot analysis localize McArdle's syndrome to chromosome 11. Science 225:57-59, 1984.
Lebo RV, Anderson LA, Lau Y-F, Flandermeyer R, Kan YW: Flow sorting analysis of normal and abnormal human genomes. Cold Spring Harbor Symposium, New York, 51:169-176, 1986.
Maniatis T, Fritsch EF, Sambrook J. Molecular Cloning: A Laboratory Manual, 1st Edition, Cold Spring Harbor Laboratory Press, 1982.
Milunsky JM, Lebo RV, Ikuta T, Maher TA, Haverty CE, Milunsky A. Mutation Analysis in Rett Syndrome. Genetic Testing 5(4):321-325, 2001.
Riordan et al., "Identification of the cystic fibrosis gene: cloning and characterization of complementary DNA. Science 245:1066-1073, 1989.
Sambrook J, Fritsch EF, Maniatis T. Molecular Cloning: A Laboratory Manual, Vols. 1-3, 2nd Edition, Cold Spring Harbor Laboratory Press, 1989.
Wang Z, Milunsky JM, Yamin M, MaherTA, Oates R, Milunsky A. Analysis of 100 cystic fibrosis mutations in 92 patients with congenital absence of the vas deferens by mass spectrometry. Am J Human Genetics 69(4)210, 2001.

## Claims

1. A molecular genetic testing method, comprising the steps of: synthesizing a DNA molecule for use as a positive test control by incorporating three or more selected sequence changes from any genomic location into one or more selected target DNA sequences using template polynucleotides from one or many individuals, comprising incorporating oligonucleotide primers comprising selected polynucleotide sequences into the target DNA sequence either by PCR amplification or site directed mutagenesis, and assuring that all selected sequence changes, being mutations or polymorphisms, are present in the synthesized positive test control to minimize the number of control reactions or to simplify dispensing and selection of control aliquots in subsequent DNA assays.

2. The method of claim 1, further comprising the steps of:
utilizing the prepared positive test controls with three or more sequence changes each in subsequent DNA assays to simplify assay design and set up by using a control with multiple allelic sequences or by adding the same control prepared for multiple sites being tested.

3. The method of claim 1, further comprising the step of: ligating the synthesized adjacent regions of the same gene together in any order.

## Patentansprüche

1. Molekulargenetisches Testverfahren, umfassend folgende Schritte:
Synthetisieren eines DNS-Moleküls zur Verwendung als positive Testkontrolle durch Aufnahme von drei oder mehr ausgewählten Sequenzänderungen von einer beliebigen Genomstelle in eine oder mehr ausgewählte Ziel-DNS-Sequenzen unter Verwendung von Templat-Polynukleotiden von einem oder mehr Individuen, umfassend die Aufnahme von Oligonukleotid-Primern, welche ausgewählte Polynukleotidsequenzen umfassen, in der Ziel-DNS-Sequenz entweder durch PCR-Amplifikation oder ortsgerichtete Mutagenese und Sicherstellen, dass sämtliche ausgewählten Sequenzänderungen, ob es sich um Mutationen oder Polymorphismen handelt, in der synthetisierten positiven Testkontrolle vorhanden sind, um die Anzahl Kontrollreaktionen zu minimieren oder um die Abgabe und Auswahl von Kontroll-Aliquoten bei anschließenden DNS-Assays zu vereinfachen.

2. Verfahren nach Anspruch 1, welches ferner folgende Schritte umfasst:
Verwenden der dargestellten positiven Testkontrollen mit je drei oder mehr Sequenzänderungen bei anschließenden DNS-Assays, um den Aufbau und die Anordnung der Assays zu vereinfachen durch Verwenden einer Kontrolle mit multiplen Allelsequenzen oder durch Hinzufügen der gleichen Kontrolle, die für mehrere einem Test unterzogene Stellen dargestellt wurde.

3. Verfahren nach Anspruch 1, welches ferner folgenden Schritt umfasst:
Ligieren der synthetisierten benachbarten Regionen desselben Gens in einer beliebigen Reihenfolge.

## Revendications

1. Procédé de test génétique moléculaire, comprenant les étapes suivantes :
synthétiser une molécule d'ADN destinée à être utilisée comme témoin de test positif en incorporant trois ou plus de trois changements de séquence sélectionnés à partir de tout emplacement génomique dans une ou plusieurs séquences d'ADN cible sélectionnées en utilisant des polynucléotides matrices provenant d'un ou
plusieurs individus, comprenant l'incorporation dans la séquence d'ADN cible d'amorces oligonucléotidiques comprenant des séquences polynucléotidiques sélectionnées, par amplification PCR ou mutagénèse dirigée sur un site, en s'assurant que tous les changements de séquence sélectionnés, qu'il s'agisse de mutations ou de polymorphismes, sont présents dans le témoin de test positif synthétisé pour minimiser le nombre de réactions de témoin ou simplifier la distribution et la sélection de quantités aliquotes de témoin dans des essais d'ADN ultérieurs.

2. Procédé selon la revendication 1, comprenant de plus les étapes suivantes :
utiliser les témoins de test positif préparés avec trois ou plus de trois séquences chacun dans des essais d'ADN ultérieurs pour simplifier la conception et la mise en oeuvre de l'essai en utilisant un témoin ayant de multiples séquences alléliques ou en ajoutant le même témoin préparé pour de multiples sites étant testés.

3. Procédé selon la revendication 1, comprenant de plus l'étape suivante :
ligaturer ensemble les régions adjacentes synthétisées, dans un ordre quelconque.
